# EUROPEAN PATENT APPLICATION

(11) **EP 4 840 455 A1**
(43) Date of publication of application: **18.11.2026**
(21) Application number: 25738634.2
(22) Date of filing: 10.01.2025
(51) Int. Cl.: A61K 35/744, A61P 1/02, C12N 1/20, C12R 1/46

(54) **ORAL PROBIOTIC**

(30) Priority: 11.01.2024 ES 202430023
(71) Applicant: Darwin Bioprospecting Excellence S.L., 46980 Paterna (Valencia) (ES); Fundación para el Fomento de la Investigación Sanitaria y Biomédica de la Comunitat Valenciana (FISABIO), 46020 Valencia (ES)
(72) Inventor: MIRA OBRADOR, Alejandro, 46020 Valencia Valencia (ES); ADRADOS PLANELL, Ana, 46020 Valencia Valencia (ES); CARDA DIÉGUEZ, Miguel, 46020 Valencia Valencia (ES); PORCAR MIRALLES, Manuel, 46980 Paterna Valencia (ES); VILANOVA SERRADOR, Cristina, 46980 Paterna Valencia (ES); PASCUAL MARTÍNEZ, Francisco Javier, 46980 Paterna Valencia (ES); GIMENO VALERO, Helena Amalia, 46980 Paterna Valencia (ES)
(74) Representative: Cueto, Sénida
(86) International application number: PCT/ES2025/070007
(87) International publication number: WO 2025/149699

(57) **Abstract**

The present invention relates to a bacterial strain of *Streptococcus salivarius* with oral probiotic properties and a minimum inhibitory concentration of at least 500 ppm.

## Description

### Field of the invention

The invention relates to strains of *Streptococcus salivarius* and their use as oral probiotics for the production of essential amino acids.

### Background

Humans need to obtain essential amino acids (EAs) from external sources, as our bodies are unable to synthesise them, or do so at a rate that is too low to allow for optimal growth and function. These AEs, traditionally obtained through the diet, are also produced by bacteria that colonise various niches within the human body, with a particularly significant contribution coming from bacteria associated with the digestive system and, more specifically, the mouth. AEs play an important role in regulating metabolism and immune system functions, whilst also acting as neurotransmitters or as precursors to them, amongst other functions. A decline in amino acid-producing bacteria due to changes in dietary habits, an ageing population or the excessive use of antibiotics can have a negative effect on human health. The use of probiotics capable of restoring this capacity for amino acid production may provide support in the treatment and prevention of diseases with significant social and economic impact.

An alternative to the use of oral probiotics to address essential amino acid deficiencies is the intake of amino acids in the form of dietary supplements. The drawback of this strategy is that the absorption of essential amino acids is limited to the intestine, thereby bypassing the contribution from the oral cavity via mucosal permeability. Furthermore, the amounts provided by supplements are usually equal to or greater than the recommended daily intake and are generally taken in a single daily dose, whereas synthesis by a microorganism is lower, albeit sustained over time, including during periods of fasting.

The *Streptococcus salivarius* strains disclosed by this invention are capable of producing both essential and non-essential L-amino acids and secreting them into the medium in free form. This means that they can be immediately incorporated into human metabolism through direct absorption in the oral cavity, or transported with saliva to the intestine, where they are absorbed alongside the amino acids released following the lysis of the bacteria that takes place in the stomach.

Document EP1483366B1 discloses a strain of *S. salivarius* designated M18; but, it does not disclose the use of this strain for the production of AEs. Furthermore, the strains of the present invention exhibit better probiotic properties than that strain.

The *S. salivarius* strains currently marketed as probiotics for oral health (M18 and K12) have not been described as producing essential amino acids. *Streptococcus salivarius* K12 is characterised by the production of the bacteriocins *salivaricin* A2 and *salivaricin B*. Meanwhile, the reference strain M18 produces four bacteriocins-*salivaricins* A2, 9, MPS and M-which act against the cariogenic bacterium *Streptococcus mutans*. Furthermore, it produces dextranases and ureases, which help to reduce the accumulation of dental plaque and the acidification that causes caries. In the present invention, we further demonstrate through comparative experiments that the prior-art strains exhibit inferior or even adverse probiotic capabilities compared to the *S. salivarius* strains covered by the invention, such as a reduced ability to produce AEs, lower resistance to fluoride and greater acidogenic capacity.

### Description of the invention

The expression 'a strain of the invention', as used herein, is a collective term referring to any strain of *S. salivarius* described herein and its derivative strains.

One object of the invention relates to an oral probiotic strain of *Streptococcus salivarius* which:
(a) comprises a 16S rRNA-encoding gene that has ≥95% sequence identity to SEQ ID NO: 1, 2 or 3;
(b) is *Streptococcus salivarius* CDWN00916 deposited in the Spanish Type Culture Collection (CECT) under accession number CECT 30898,
(c) is Streptococcus salivarius CDWN00918 deposited in the Spanish Type Culture Collection (CECT) under accession number CECT 30899, or
(d) is Streptococcus salivarius CDWN00921, deposited in the Spanish Type Culture Collection (CECT) under accession number CECT 30900,
that has a minimum inhibitory concentration to fluoride ions of at least 500 ppm

In a particular embodiment, the 16S rRNA sequence has at least 96%, 97%, 98%, 99%, or 100% identity to the sequence of one or more of SEQ ID NO: 1, 2, or 3;

A bacterial strain of the invention may comprise a 16S rRNA-encoding gene that has ≥95% (i.e., equal to or higher than 95%), ≥96%, ≥97%, ≥98%, ≥99%, or 100% sequence identity to the 16S rRNA-encoding gene of *S. salivarius* CECT 30898 (SEQ ID NO: 1). For example, the 16S rRNA-encoding gene may have ≥98.4%, ≥98.5%, ≥98.6%, ≥98.7%, ≥98.8%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to SEQ ID NO: 1. The 16S rRNA-encoding gene of the bacterial strain of the invention may differ from SEQ ID NO: 1 by 1, ≤2 (i.e., 2 or fewer), ≤3, ≤4, ≤5, ≤6, ≤7, ≤8, ≤9, ≤10, ≤15, ≤20, ≤25, or ≤29 bases. The bacterial strain may be a strain of *S. salivarius*.

A bacterial strain of the invention may comprise a 16S rRNA-encoding gene that has ≥95% (i.e., equal to or higher than 95%), ≥96%, ≥97%, ≥98%, ≥99%, or 100% sequence identity to the 16S rRNA-encoding gene of *S. salivarius* CECT 30899 (SEQ ID NO: 2). For example, the 16S rRNA-encoding gene may have ≥98.4%, ≥98.5%, ≥98.6%, ≥98.7%, ≥98.8%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to SEQ ID NO: 2. The 16S rRNA-encoding gene of the bacterial strain of the invention may differ from SEQ ID NO: 2 by 1, ≤2 (i.e., 2 or fewer), ≤3, ≤4, ≤5, ≤6, ≤7, ≤8, ≤9, ≤10, ≤15, ≤20, ≤25, or ≤29 bases. The bacterial strain may be a strain of *S. salivarius*.

A bacterial strain of the invention may comprise a 16S rRNA-encoding gene that has ≥95% (i.e., equal to or higher than 95%), ≥96%, ≥97%, ≥98%, ≥99%, or 100% sequence identity to the 16S rRNA-encoding gene of *S. salivarius* CECT 30900 (SEQ ID NO: 3). For example, the 16S rRNA-encoding gene may have ≥98.4%, ≥98.5%, ≥98.6%, ≥98.7%, ≥98.8%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to SEQ ID NO: 3. The 16S rRNA-encoding gene of the bacterial strain of the invention may differ from SEQ ID NO: 3 by 1, ≤2 (i.e., 2 or fewer), ≤3, ≤4, ≤5, ≤6, ≤7, ≤8, ≤9, ≤10, ≤15, ≤20, ≤25, or ≤29 bases. The bacterial strain may be a strain of *S. salivarius*.

In a particular embodiment, the 16S rRNA sequence identity is at least 97%, 98%, 99%, or 100% with one or more of SEQ ID NO: 2 or 3;

An object of the invention relates to an oral probiotic strain of *Streptococcus salivarius* that:
(a) comprises a 16S rRNA-encoding gene that has ≥95% sequence identity to SEQ ID NO: 1, 2, or 3;
(b) comprises a genome that has ≥90% identity to the genome of *S. salivarius*, CDWN00916, CDWN00918, or CDWN00921;
(b) is *Streptococcus salivarius* CDWN00916 deposited in the Spanish Type Culture Collection (CECT) under accession number CECT 30898,
(c) is *Streptococcus salivarius* CDWN00918 deposited in the Spanish Type Culture Collection (CECT) under accession number CECT 30899, or
(d) is *Streptococcus salivarius* CDWN00921, deposited in the Spanish Type Culture Collection (CECT) under accession number CECT 30900,
that has a minimum inhibitory concentration to fluoride ions of at least 500 ppm.

The genomes of strains CDWN00916, CDWN00918, and CDWN00921 are available in the GenBank database under accession numbers JAXKQD000000000, JAXKQE000000000, and JAXKLG000000000, respectively.

A bacterial strain of the invention may comprise a genome having ≥90% (i.e., equal to or higher than 90%), ≥91%, ≥92%, ≥93%, ≥94%, ≥95%, ≥96%, ≥97%, ≥98%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to the genome sequence of *S. salivarius* CDWN00916, GenBank ID JAXKQD000000000. The genome of said strain is understood to be the totality of the sequences or "contigs" listed for GenBank: JAXKQD000000000.1

A bacterial strain of the invention may comprise a genome having ≥90% (i.e., equal to or higher than 90%), ≥91%, ≥92%, ≥93%, ≥94%, ≥95%, ≥96%, ≥97%, ≥98%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to the genome sequence of *S. salivarius* CDWN00918, GenBank ID JAXKQE000000000. The genome of said strain is understood to be the totality of the sequences or "contigs" listed for GenBank: J JAXKQE000000000.1

A bacterial strain of the invention may comprise a genome having ≥90% (i.e., equal to or higher than 90%), ≥91%, ≥92%, ≥93%, ≥94%, ≥95%, ≥96%, ≥97%, ≥98%, ≥99%, ≥99.1%, ≥99.2%, ≥99.3%, ≥99.4%, ≥99.5%, ≥99.6%, ≥99.7%, ≥99.8%, ≥99.9%, or 100% sequence identity to the genome sequence of *S. salivarius* CDWN00921, GenBank ID JAXKLG000000000. The genome of this strain is defined as the totality of the sequences or "contigs" listed for GenBank: JAXKLG000000000.1

Herein "oral probiotic" means one or more strains of *S salivarius* beneficial for oral use, which do not come from a sample of respiratory, oral, or digestive tract tissue of a human subject with pathologies, preferably inflammatory pathologies and whose beneficial characteristics are its antimicrobial activity, effect on pH, essential amino acid production capacity and aid in the digestion of dairy products.

The microorganism strains of the present invention surprisingly exhibit the characteristic of having a high resistance to the fluoride ion. The resistance of oral probiotics to fluoride is important to prevent their viability from being affected as a consequence of tooth brushing or with the use of mouthwashes. Surprisingly, the strains of the invention exhibit increased resistance to fluoride-derived compounds usually present in oral hygiene products compared to prior art strains. This allows the beneficial oral effects to last longer as it will not be necessary to take another dose of probiotics after, for example, brushing which may remove other probiotics from the oral cavity. The usual concentration of fluoride in toothpastes is 1500 ppm, saliva produces a dilution effect of about 1/3 to about 1/4, resulting in about 500 ppm. As will be shown below, strains M18 and K12 have a Minimum Inhibitory Concentration (MIC) of ppm sodium fluoride (NaF) while that of the strains of the present invention is superior.

In a particular embodiment the fluoride ion is sodium fluoride

In a particular embodiment the *S. salivarius* strains of the invention have a minimum inhibitory concentration to the fluoride ion of at least 250 or 251 or 252 or 253 or 254 or 255 or 256 or 257 or 258 or 259 or 260 or 261 or 262 or 263 or 264 or 265 or 266 or 267 or 268 or 269 or 270 or 271 or 272 or 273 or 274 or 275 or 276 or 277 or 278 or 279 or 280 or 281 or 282 or 283 or 284 or 285 or 286 or 287 or 288 or 289 or 290 or 291 or 292 or 293 or 294 or 295 or 296 or 297 or 298 or 299 or 300 or 301 or 302 or 303 or 304 or 305 or 306 or 307 or 308 or 309 or 310 or 311 or 312 or 313 or 314 or 315 or 316 or 317 or 318 or 319 or 320 or 321 or 322 or 323 or 324 or 325 or 326 or 327 or 328 or 329 or 330 or 331 or 332 or 333 or 334 or 335 or 336 or 337 or 338 or 339 or 340 or 341 or 342 or 343 or 344 or 345 or 346 or 347 or 348 or 349 or 350 or 351 or 352 or 353 or 354 or 355 or 356 or 357 or 358 or 359 or 360 or 361 or 362 or 363 or 364 or 365 or 366 or 367 or 368 or 369 or 370 or 371 or 372 or 373 or 374 or 375 or 376 or 377 or 378 or 379 or 380 or 381 or 382 or 383 or 384 or 385 or 386 or 387 or 388 or 389 or 390 or 391 or 392 or 393 or 394 or 395 or 396 or 397 or 398 or 399 or 400 or 401 or 402 or 403 or 404 or 405 or 406 or 407 or 408 or 409 or 410 or 411 or 412 or 413 or 414 or 415 or 416 or 417 or 418 or 419 or 420 or 421 or 422 or 423 or 424 or 425 or 426 or 427 or 428 or 429 or 430 or 431 or 432 or 433 or 434 or 435 or 436 or 437 or 438 or 439 or 440 or 441 or 442 or 443 or 444 or 445 or 446 or 447 or 448 or 449 or 450 or 451 or 452 or 453 or 454 or 455 or 456 or 457 or 458 or 459 or 460 or 461 or 462 or 463 or 464 or 465 or 466 or 467 or 468 or 469 or 470 or 471 or 472 or 473 or 474 or 475 or 476 or 477 or 478 or 479 or 480 or 481 or 482 or 483 or 484 or 485 or 486 or 487 or 488 or 489 or 490 or 491 or 492 or 493 or 494 or 495 or 496 or 497 or 498 or 499 or 500 or 501 or 502 or 503 or 504 or 505 or 506 or 507 or 508 or 509 or 510 or 511 or 512 or 513 or 514 or 515 or 516 or 517 or 518 or 519 or 520 or 521 or 522 or 523 or 524 or 525 or 526 or 527 or 528 or 529 or 530 or 531 or 532 or 533 or 534 or 535 or 536 or 537 or 538 or 539 or 540 or 541 or 542 or 543 or 544 or 545 or 546 or 547 or 548 or 549 or 550 ppm of fluoride ions.

In a particular embodiment, the *S. salivarius* strains of the invention have a minimum inhibitory concentration to fluoride ions of at least 550 ppm, preferably at least 600 ppm, more preferably at least 700 ppm, most preferably between 700 and 1500 ppm in an in vitro experiment, even more preferably between 800 and 1200 ppm, and most preferably between 850 and 1100 ppm or between 900 and 1000 ppm of NaF.

In another specific embodiment, the MIC of the strains of the invention is at least 600 ppm of NaF, preferably at least 700 ppm of NaF, more preferably at least 800 ppm of NaF, and even more preferably at least 900 ppm of NaF.

As indicated above, high resistance to fluoride ions is a beneficial characteristic of an oral probiotic.

In a particular embodiment, the strain of the invention exhibits one or more properties of an oral probiotic in addition to fluoride resistance

The "oral probiotic properties" are selected from one or more of the following: production of essential amino acids, antimicrobial activity against oral pathogens, reduced acidogenic capacity compared to prior art strains-preferably strains M18 and K12-and aid in the digestion of dairy products.

In a particular embodiment, the EAs produced by the strains of the invention in greater quantities than the prior art strains are one or more of the following: histidine, isoleucine, leucine, lysine, methionine, phenylalanine, threonine, tryptophan, and valine.

In a particular embodiment, the essential amino acids are: isoleucine, leucine, tryptophan, phenylalanine + tyrosine, threonine, valine, and methionine + cysteine.

In another particular embodiment, the essential amino acids are: isoleucine, leucine, phenylalanine + tyrosine, threonine, and valine.

In another particular embodiment, the essential amino acids are: leucine, valine, and phenylalanine.

A reduced acidogenic activity in oral bacteria is of great importance in the field of health, as it can lead to acidification that favors the development of caries and, in turn, promotes the proliferation of other acidogenic and acid-tolerant microorganisms, including species such as *Streptococcus mutans* and *Lactobacillus spp*., among others. This leads to rapid fermentation of dietary sugars, resulting in a decrease in pH and, consequently, demineralization of the teeth, triggering caries. As will be seen below, the strains of the invention exhibit reduced acidogenic activity compared to prior art strains.

In general, the number of *S. salivarius* cells of the invention administered to an individual will range from approximately 10² to 10¹⁵ colony-forming units (CFU), preferably from about 10³ to 10¹⁴ CFU, more preferably from about 10⁵ to 10¹² CFU, typically around 10⁷ to 10¹⁰ CFUs per dose. This dose may comprise one or more of the strains of the invention.

In a particular embodiment, the strain of the invention may be present in its live form as in an inactivated form, that is, as a postbiotic.

Postbiotics are bioactive compounds derived from probiotic microorganisms, generated through their metabolism or released following cell lysis. These include metabolites, cellular components, and other beneficial products that have positive effects on the host's health. Unlike probiotics, postbiotics do not contain live microorganisms, which can facilitate their storage, transport, and administration. Their benefits include modulation of the immune system, strengthening of the intestinal barrier, and the provision of bioactive compounds such as amino acids, short-chain fatty acids, and antimicrobial peptides.

In one particular embodiment, *Streptococcus salivarius* strains may undergo an inactivation process (e.g., by heat or pressure) to produce a bacterial lysate, thereby obtaining a postbiotic. The lysate contains bioactive components released during the rupture of the bacterial cell and those attached to the cell wall or in the immediate vicinity of the bacterium, including specific metabolites and cellular fragments that retain the ability to provide essential amino acids. The use of the strain as a postbiotic offers additional advantages in terms of product stability, transport, and safety, as it eliminates the risk associated with the viability of live microorganisms.

The lysate or postbiotic is obtained by subjecting the strain of the invention to a controlled cell lysis process, which may include thermal, mechanical, or chemical treatments; any process frequently used in the field and known to a person skilled in the art is suitable. This process ensures the preservation of the bioactive compounds responsible for the aforementioned benefits, while also ensuring the elimination of viable microorganisms. Subsequent processing may include the concentration of the inactivated strain and/or the supernatant, as well as its possible freeze-drying or spray-drying.

As will be shown in the examples, the strains of the invention can improve a subject's digestion of dairy products and dairy derivatives, since a study of the genome of these strains has found that they possess genes involved in the metabolism of lactose and galactose that are not present in other strains of the prior art. This additional advantage would be particularly useful for individuals who are lactose intolerant.

In a particular embodiment, the *S. salivarius* strains of the invention contain the EC 3.2.1.23 (B-galactosidase) gene and/or the lactose/galactose permease gene in their genome. A person skilled in the art will understand that the sequence of these genes in the genome of the *S. salivarius* strains may differ from the canonical sequence recognized for *S. salivarius* but that, in any case, it will result in a functional, active protein.

An additional object of the invention relates to a strain of *Streptococcus salivarius* deposited in the Spanish Type Culture Collection under accession number CECT 30898, or strains derived thereof, wherein said derived strains have the same, better, or inferior oral probiotic properties than strain CECT 30898.

This strain was deposited on July 25, 2023, in the Spanish Type Culture Collection, Calle Catedrático Agustín Escardino, 9, CP46980, Paterna (Valencia), Spain, in accordance with the provisions of the Budapest Treaty.

In the present invention, the strain CECT 30898 and CDWN00916 are interchangeable terms and are used indistinctly.

Variants of this strain refer to strains derived from CECT 30898 or obtained from CECT 30898, and may have mutations compared to *S. salivarius*. Preferably, the derived strain has the same, better, or inferior oral probiotic properties as the original strain CECT 30898. Preferably, the strain derived from CECT 30898 possesses at least 80% or more, preferably at least 90% or more, more preferably at least 95% or more, or even up to 100% or more of the oral probiotic properties compared to the CECT 30898 strain under identical conditions.

In a particular embodiment, the number of *S. salivarius* cells of strain CDWN00916 administered to the individual will be at least approximately 10⁹ CFU, or approximately 10⁹ to 10¹² colony-forming units (CFU). This dose is sufficient to provide a subject with the recommended daily amount of essential amino acids: Ile, Leu, Phe/Tyr, Thr, Val, Trp, and Met/Cys

The present invention also relates to a strain of *Streptococcus salivarius* deposited in the Spanish Type Culture Collection under accession number CECT 30899 or strains derived thereof, wherein said derived strains have the same, better, or inferior properties as an oral probiotic compared to strain CECT 30899. This strain was deposited on July 25, 2023, in the Spanish Type Culture Collection.

In the present invention, the strain CECT 30899 and CDWN00918 are interchangeable terms and are used indistinctly.

Variants of this strain refer to strains derived from CECT 30899 or obtained from CECT 30899, and may have mutations compared to *S. salivarius*; preferably, the derived strain has the same, better, or inferior oral probiotic properties as the original CECT 30899 strain. Preferably, the strain derived from CECT 30899 possesses at least 80% or more, preferably at least 90% or more, more preferably at least 95% or more, or even up to 100% or more of the oral probiotic properties compared to the CECT 30899 strain under identical conditions.

In a particular embodiment, the number of *S. salivarius* cells of strain CDWN00918 administered to the individual will be at least approximately 10⁹ CFU, or approximately 10⁹ to 10¹¹ colony-forming units (CFU). This dose is sufficient to provide a subject with the recommended daily amount of essential amino acids: Ile, Leu, Phe/Tyr, Thr, and Val.

The present invention also relates to a strain of *Streptococcus salivarius* deposited in the Spanish Type Culture Collection under accession number CECT 30900, or strains derived thereof, wherein said derived strains have the same, better, or inferior oral probiotic properties than strain CECT 30900. This strain was deposited on July 25, 2023, in the Spanish Type Culture Collection.

In the present invention, the strain CECT 30900 and CDWN00921 are interchangeable terms and are used indistinctly.

Variants of this strain refer to strains derived from CECT 30900 or obtained from CECT 30900, and may have mutations compared to *S. salivarius*; preferably, the derived strain has the same, better, or inferior oral probiotic properties than the original CECT 30900 strain. Preferably, the strain derived from CECT 30900 possesses at least 80% or more, preferably at least 90% or more, more preferably at least 95% or more, or even up to 100% or more of the oral probiotic properties compared to the CECT 30900 strain under identical conditions.

In a particular embodiment, the number of *S. salivarius* cells of strain CDWN00921 administered to the individual will be at least approximately 10⁷ CFU, or approximately 10⁷ to 10⁹ colony-forming units (CFU). This dose is sufficient to provide a subject with the recommended daily amount of essential amino acids: Ile, Leu, Phe/Tyr, Thr, and Val.

Variants or strains derived from the strains of the invention typically retain the oral probiotic properties of their corresponding original strain.

Based on the foregoing, the advantages of the strains of the present invention are:
1. Exploitation of the natural properties of the oral commensal microbiota.
2. Due to their ability to produce essential amino acids, these strains represent a preventive strategy against the development of pathologies associated with a deficiency of these amino acids.
3. Sustained production and release of amino acids over time, even during fasting periods.
4. The strains also exhibit antimicrobial activity, which provides an additional benefit to oral health by inhibiting the growth of pathogenic oral microorganisms involved in the development of certain oral diseases such as dental caries and periodontal disease.
5. The strains are more resistant to fluoride compounds than those of the prior art, making them more resistant to oral hygiene practices, including brushing with fluoride toothpaste. This ensures that the administered probiotic bacteria are not affected by oral hygiene practices.
6. The strains exhibit lower acidogenic capacity than those of the prior art, which results in a reduced ability to induce dental caries.

Another additional object of the invention relates to active extracts obtained from the *S. salivarius* strains of the invention described above.

An active extract is defined as a concentrated mixture of bioactive compounds obtained from an organism, cell, or tissue through specific extraction and purification processes. These compounds are responsible for exerting specific biological effects, such as antimicrobial activity, immunomodulation, nutritional support, or the enhancement of physiological functions. Active extracts may contain secondary metabolites, peptides, proteins, lipids, amino acids, polysaccharides, and other components with functional properties, generally in a more concentrated form.

These active extracts can be used in a similar manner in formulations and therapeutic applications. The extracts can be obtained by following protocols known in the art, typically through cell culture and centrifugation and/or microfiltration.

This extract is rich in essential amino acids and also comprises other compounds with antimicrobial activity.

Another additional object of the invention relates to a probiotic formulation for oral administration comprising one or more of the strains listed above, or the extract of the invention.

In a particular embodiment, the probiotic formulation comprises equal amounts of CDWN00916, CDWN00918, and CDWN00921 strains.

In another particular embodiment, the probiotic formulation comprises the CDWN00916, CDWN00918, and CDWN00921 strains in a ratio of approximately 1:2:1 to 1:3:1.

In another specific embodiment, the probiotic formulation comprises the CDWN00918, CDWN00916, and CDWN00921 strains, in a ratio of approximately 1:2:1 to 1:3:1

In another specific embodiment, the probiotic formulation comprises the CDWN00916, CDWN00921, and CDWN0018 strains, in a ratio of approximately 1:2:1 to 1:3:1

In a further embodiment, the probiotic formulation for oral administration comprises:
at least one pharmaceutically acceptable carrier, diluent, and/or excipient and/or
at least one active agent

Formulations for oral administration may include powders or granules, suspensions or solutions in water or non-aqueous media, capsules, sachets, or tablets. Thickeners, flavorings, diluents, emulsifiers, dispersing aids, or binders may be desirable.

A "carrier, diluent, and/or excipient" means a carrier for delivering a strain of *S. salivarius* or an extract of the invention to an individual, wherein the carrier is compatible with the viability of the bacterial cell or the activity of the extract. Acceptable carriers suitable for use in the administration of viable *S. salivarius* strains and extracts of the invention are well known to those skilled in the art. Suitable carriers are generally inert and may be solid or liquid.

"Pharmaceutically acceptable" means a material that is not biologically or otherwise undesirable; that is, the material may be administered to a subject together with the selected compound without causing any undesirable biological effects or interacting deleteriously with any of the other components of the pharmaceutical composition in which it is contained.

Pharmaceutically acceptable carriers, diluents, and/or excipients include, but are not limited to, water, buffered saline solutions (e.g., phosphate-buffered saline solution), pharmaceutically acceptable culture media (e.g., Brain Heart Infusion (BHI), Tryptic Soy Agar (TSA), Blood Agar, etc.), or other solutions that maintain the viability of the bacteria or the activity of the extract.

In addition, such pharmaceutically acceptable carriers may be aqueous or non-aqueous solutions, suspensions, and emulsions. A variety of pharmaceutically acceptable carriers suitable for the oral administration of viable or lyophilized bacteria are well known in the art. Suitable solid carriers known in the art include, for example, magnesium carbonate; magnesium stearate; celluloses; talc; sugars such as fructose, sucrose, mannitol, and lactose; starches; flours; skim milk; and similar edible powders, but are not limited to these. Similarly, carriers for the administration of extracts are well known.

Typical diluents, by way of example, include: starches; lactose; mannitol; kaolin; calcium phosphate or sulfate; inorganic salts such as sodium chloride; and powdered sugars or cellulose.

The compositions may also include excipients such as compression aids; resins; fillers; binders; lubricants; solvents; anti-caking agents; disintegrants; preservatives; buffers; flavorings; colorants; sweeteners; and fragrances, as appropriate.

Typical binders include starch; gelatin; sugars, such as lactose, fructose, and glucose; and the like. Natural and synthetic gums are also suitable, including arabic gum, alginates; methylcellulose; polyvinylpyrrolidone; tragacanth; and the like. Polyethylene glycol, ethylcellulose, and waxes may also serve as binders.

Lubricants to prevent adhesion to the matrix during formation include slippery solids such as talc, silica, magnesium stearate and calcium stearate, polyethylene glycol, stearic acid, and hydrogenated vegetable oils.

Disintegrants are substances that swell when moistened, breaking up the tablet and releasing *S. salivarius* or the extract. Disintegrants include starches; clays; celluloses; alginates and gums; more specifically, corn and potato starches; methylcellulose; agar; bentonite; wood cellulose; cation exchange resins; alginic acid; guar gum; citrus pulp; carboxymethylcellulose; powdered sponge; and sodium lauryl sulfate.

In a particular embodiment, the strain of the invention or the composition comprising it is administered as food, a beverage, or a nutraceutical. The active strain(s) or extract(s) may also be administered in the form of a mouthwash, oral rinse, toothpaste, oral spray, gargle, capsule, lozenge, syrup, dental floss, chewing gum, or chewable tablet, among other possible formulations.

In one embodiment, the edible material or beverage is a dairy-based food or beverage, including, by way of example, yogurt, cheese, milk, powdered milk, milk cookies, and flavored milks.

In a particular embodiment, the oral administration composition of *S. salivarius* is a mixture of freeze-dried *S. salivarius* strains with skim milk powder or the like, which has been flavored to enhance its pleasant taste. It is preferably presented as lozenges, chewable tablets, or capsules. A lozenge according to the invention comprises a strain of *S. salivarius* or an extract of the invention, isomalt, and EMDEX^{®}. The lozenge can be prepared by direct compression, wet granulation, or dry granulation. The lozenges can be coated according to well-known pharmaceutical practice.

The probiotic composition may additionally contain nutrients or preservatives to maintain the viability of the bacterium(s) in the formulation. As noted above, the formulation may also contain flavoring agents, coloring agents, fragrances, or other compounds that enhance the pleasant taste of the composition and/or improve patient acceptance without compromising the efficacy of the formulation. Methods for preparing formulations for oral administration are well known in the art.

In a particular embodiment, the composition of the invention may further comprise one or more active agents, which may be another strain of *S. salivarius* from among those previously defined or a different strain, or which may be non-microorganisms such as xylitol, fluoride, Manuka honey, and tannins.

In a particular embodiment, the probiotic formulation for oral administration comprises the CDWN00916 and CDWN00921 strains.

In another particular embodiment, the probiotic composition comprises the *S. salivarius* CDWN00916 and CDWN00921 strains at an appropriate concentration of CFUs to provide an individual with the necessary daily amount of essential amino acids.

In a particular embodiment, the essential amino acids are isoleucine, leucine, tryptophan, phenylalanine + tyrosine, threonine, valine, and methionine + cysteine.

In another particular embodiment, the essential amino acids are: leucine, valine, and phenylalanine.

In another particular embodiment, the formulation also comprises CDWN00918 strain.

An expert would be able to determine the amount of essential amino acids required for an individual, based on their weight and health status, by consulting, for example, the article "Joint FAO/WHO/UNU Expert Consultation on Protein and Amino Acid Requirements in Human Nutrition" (2002: Geneva, Switzerland), published by the Food and Agriculture Organization of the United Nations, the World Health Organization, and the United Nations University (2007). *Protein and Amino Acid Requirements in Human Nutrition: Report of a Joint FAO/WHO/UNU Expert Consultation*, or other similar documents, and from there determine the required concentration of CFUs of the S*. salivarius* strains.

In another particular embodiment, the probiotic composition for oral administration of the invention comprises approximately 10⁵ to 10¹² CFU, preferably 10⁸ to 10¹⁰ CFU, of one or more of the strains defined above.

Another additional object of the invention relates to the use of a strain described above or an orally administered composition for the production of essential amino acids.

In a particular embodiment, the essential amino acids are isoleucine, leucine, tryptophan, phenylalanine + tyrosine, threonine, valine, and methionine + cysteine.

In another particular embodiment, the essential amino acids are: leucine, valine, and phenylalanine.

This is especially relevant in situations of essential amino acid deficiency, as well as in situations where one wishes to supplement the intake of essential amino acids obtained from the diet.

Another additional object of the invention relates to a strain of *S. salivarius* selected from CDWN00916, CDWN00918, and CDWN00921, or an extract or formulation comprising the same as defined above, for use in the prevention or treatment of oral diseases.

In a particular embodiment, the oral diseases are selected from dental caries and periodontal diseases.

These oral diseases may be caused wholly or partially by pathogenic microorganisms.

In this document, the term "preventing" refers to administering a strain or a probiotic formulation for oral administration prior to the onset of clinical symptoms of a disease or conditions so as to prevent a physical manifestation of aberrations associated with the disease or condition. In the context of dental caries, the term "preventing" refers to administering a strain or a probiotic formulation for oral administration prior to the onset of clinical symptoms of dental caries so as to prevent a physical manifestation of aberrations associated with dental caries.

The terms "treatment" and "treating" as used herein refer to the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from dental caries, the terms "treatment" and "treating" refer to the medical management of a subject with the intent to cure, ameliorate, or stabilize the dental caries. In the context of a subject at risk of developing dental caries, the terms "treatment" and "treating" refer to the medical management of a subject with the intent to prevent the dental caries.

In one particular embodiment, dental caries is caused, at least in part, by acidogenic and/or acid-tolerant microorganisms such as *Streptococcus mutans, Scardovia wiggsiae*, and species of the *Lactobacillaceae* family, such as, for example: *Lactobacillus acidophilus, Lactobacillus casei, Lactobacillus plantarum, Lactobacillus rhamnosus*, *Lactobacillus fermentum, Lactobacillus salivarius, Lactobacillus reuteri, Lactobacillus brevis, Lactobacillus helveticus, Lactobacillus delbrueckii, and Lactobacillus bulgaricus*.

Another additional object of the invention relates to a strain of *S. salivarius* selected from CDWN00916, CDWN00918, and CDWN00921, or an extract or formulation comprising the same as defined above, for use in the prevention or treatment of dysbiosis or an imbalance of the oral microbiota.

As used herein, the term "subject" includes, among others, animals. The subject may be a vertebrate, more specifically a mammal (for example, a human, a horse, a pig, a rabbit, a dog, a sheep, a goat, a non-human primate, a cow, a cat, a guinea pig, or a rodent). The term does not denote a particular age or sex. Therefore, adult and newborn subjects, whether male or female, are covered by the present invention. A patient is a subject affected by a disease or disorder, preferably dental caries.

In a particular embodiment, the subject is an adult human, that is, 18 years of age or older.

The term "dose" refers to physically discrete units suitable as a single dose for the subject, each unit containing a predetermined amount of active material (viable *S. salivarius* or an active extract thereof), calculated to produce the desired therapeutic effect in combination with the required diluent, carrier, or excipient.

Specific dosages may vary widely depending on various individual factors, including size, weight, age, severity of the disease (e.g., the virulence and/or number of resident *S. mutans* causing dental caries), and response to therapy (e.g., the susceptibility of the individual's oral cavity to colonization or dysbiosis of the native microbiome). Methods for determining the appropriate route of administration and dosage may be determined by the dentist or another healthcare provider.

Furthermore, the invention comprises the following clauses:
1. An oral probiotic strain of *Streptococcus salivarius* that:
   (a) comprises a 16S rRNA-encoding gene that has ≥95% sequence identity to SEQ ID NO: 1, 2, or 3;
   (b) comprises a genome that has ≥90% identity to the genome of *S. salivarius*, CDWN00916, CDWN00918, or CDWN00921;
   (b) is *Streptococcus salivarius* CDWN00916 deposited in the Spanish Type Culture Collection (CECT) under accession number CECT 30898,
   (c) is *Streptococcus salivarius* CDWN00918 deposited in the Spanish Type Culture Collection (CECT) under accession number CECT 30899, or
   (d) is *Streptococcus salivarius* CDWN00921, deposited in the Spanish Type Culture Collection (CECT) under accession number CECT 30900,
   that has a minimum inhibitory concentration to fluoride ions of at least 500 ppm.
2. The strain according to the preceding clause, that has the properties of an oral probiotic: production of essential amino acids and antimicrobial activity.
3. The strain according to the preceding clause, wherein the essential amino acids are selected from one or more of the following: histidine, isoleucine, leucine, lysine, methionine, phenylalanine, threonine, tryptophan, and valine.
4. The strain according to any of the preceding clauses, wherein resistance to fluoride ions comprises an MIC of at least 550 ppm of fluoride ion.
5. The strain according to any of the preceding clauses, obtained from a respiratory or oral tissue sample from a human subject.
6. The strain according to the preceding clause, in which the subject does not have a condition that would affect the probiotic use of the strain, such as an inflammatory condition.
7. The *Streptococcus salivarius* strain according to any one of the preceding clauses 1 to 6, deposited in the Spanish Type Culture Collection under accession number CECT 30899, or strains derived thereof, wherein said derived strains have the same or lesser oral probiotic properties than strain CECT 30899.
8. The *Streptococcus salivarius* strain according to any one of the preceding clauses 1 to 6, deposited in the Spanish Type Culture Collection under number CECT 30898, or strains derived thereof, wherein said derived strains have the same or lesser oral probiotic properties than strain CECT 30898.
9. The *Streptococcus salivarius* strain according to any one of the preceding clauses 1 to 6, deposited in the Spanish Type Culture Collection under number CECT 30900, or strains derived thereof, wherein said derived strains have the same or lesser oral probiotic properties than strain CECT 30900.
10. An active extract obtained from one or more of the strains as defined in any of clauses 1 to 9.
11. A probiotic formulation for oral administration comprising one or more of the strains defined in any of clauses 1 to 9 and/or the active extract of the preceding clause.
12. The formulation according to the preceding clause comprising:
   at least one pharmaceutically acceptable carrier, diluent, and/or excipient and/or
   at least one active agent.
13. The formulation according to any of the preceding claims 11 or 12, wherein the active agent is at least one other distinct strain defined according to any of clauses 1 to 9 and/or another strain of *S. salivarius*.
14. The formulation according to any one of the preceding clauses 11 to 13, comprising the *S. salivarius* strain CECT 30898 and CECT 30900.
15. The formulation according to any one of the preceding clauses 11 to 14, which is included in a food, beverage, and/or nutraceutical.
16. The formulation according to any one of the preceding clauses 11 to 15, comprising approximately 10⁵ to 10¹² CFU, preferably 10⁸ to 10¹⁰ CFU, of one or more of the strains defined in any one of clauses 1 to 9.
17. The formulation according to the preceding clause, comprising an appropriate concentration of CFUs to provide the necessary daily amount of essential amino acids for a subject.
18. Use of one or more of the strains defined in any of clauses 1 to 9 and/or the formulation defined in any of clauses 11 to 17 for the production of essential amino acids.
19. The use as described in the preceding clause, wherein the production of essential amino acids comprises the daily amount required by a subject.
20. A strain according to any one of clauses 1 to 9, or an active extract according to clause 10, or a formulation according to any one of clauses 11 to 17, for use in the prevention or treatment of an oral disease.
21. The strain for use as described in the preceding clause, wherein the oral disease is selected from dental caries and periodontal disease.
22. The strain for use as described in the preceding clause, wherein dental caries is caused at least in part by *S. mutans*.
23. A strain according to any one of clauses 1 to 9, or an active extract according to clause 10, or a formulation according to any one of clauses 11 to 17 for use in the prevention or treatment of dysbiosis or an imbalance of the oral microbiota, including dental caries, periodontitis, and halitosis.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1 , about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

### Brief description of the figures

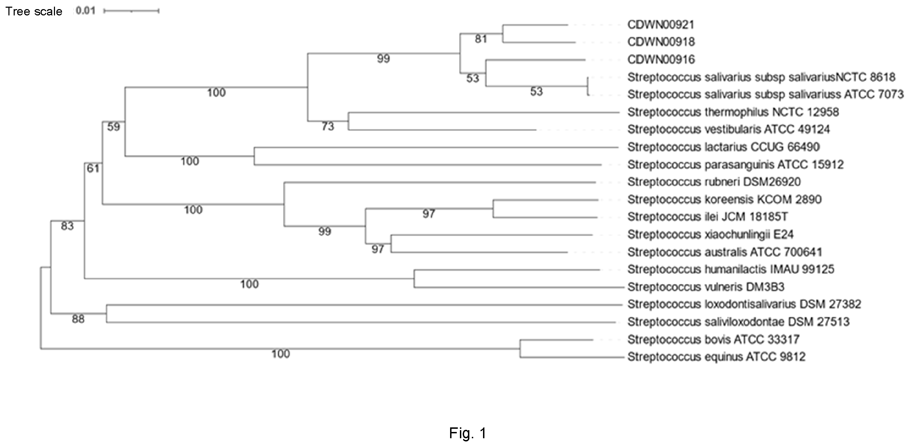

**Figure 1****:** Phylogenomic tree obtained using the *Maximum Likelihood* (ML) method based on genomic similarity distances calculated with the TYGS tool (https://tygs.dsmz.de/). The bootstrap values after 100 pseudoreplicates are shown at the nodes. Jan P. Meier-Kolthoff, Joaquim Sardà Carbasse, Rosa L. Peinado-Olarte, Markus Göker, "TYGS and LPSN: a database for fast and reliable genome-based classification and nomenclature of prokaryotes, Nucleic Acids Research, Volume 50, Issue D1, January 7, 2022, Pages D801-D807, https://doi.org/10.1093/nar/gkab902. NCTC 8616 and ATCC 7073 strains are the same.

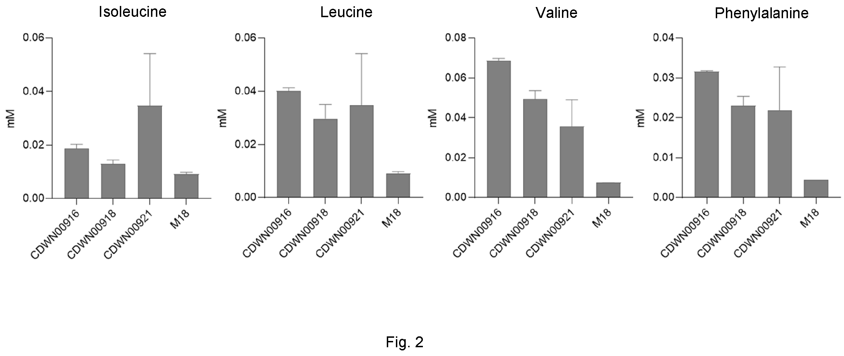

**Figure 2****:** Concentrations of essential amino acids measured by Nuclear Magnetic Resonance (NMR) in supernatants from *S. salivarius* strains CDWN00916, CDWN00918, CDWN00921, and the reference strain M18 at 37 °C for 2 hours. Mean values and standard deviations of biological duplicates are shown.

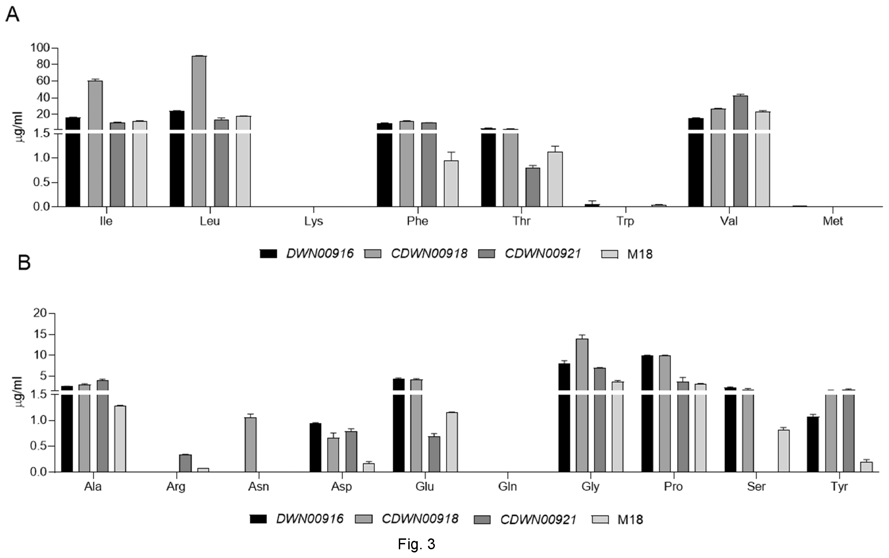

**Figure 3****:** Concentrations of (A) essential and (B) non-essential amino acids measured by UPLC in the supernatants of *S. salivarius* strains CDWN00916, CDWN00918, and CDWN00921, and the reference strain M18, in 1% (w/v) dextrose and 1% (w/v) sucrose at 37 °C under static conditions for 6 hours. Mean values and standard deviations of biological duplicates are shown.

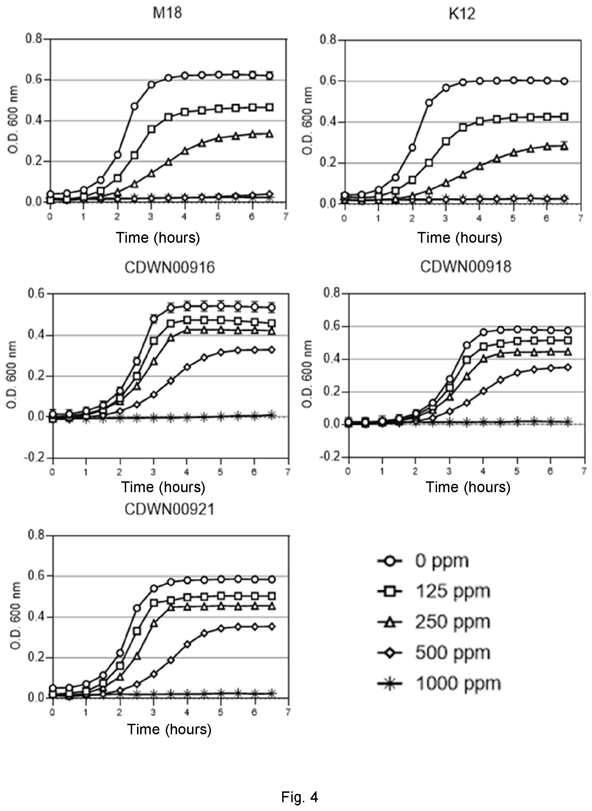

**Figure 4****:** Growth curves of the *S. salivarius* strains CDWN00916, CDWN00918, and CDWN00921 and the reference strains K12 and M18. The strains were grown on BHI until the stationary phase, after which an initial OD₆₀₀ of 0.05 was adjusted in fresh BHI medium supplemented with NaF at different concentrations (0, 125, 250, 500, 1000 ppm, parts per million). Two replicates of 200 µl from each tested condition were dispensed into a 96-well microplate, and the absorbance of the culture was measured at 600 nm using a TECAN Infinite^{®} 200 PRO plate reader. Measurements were taken every 30 minutes for 6.5 hours at 37°C. The mean net OD values, calculated after subtracting the corresponding blank value, are shown for each strain under the tested conditions according to the experimental design.

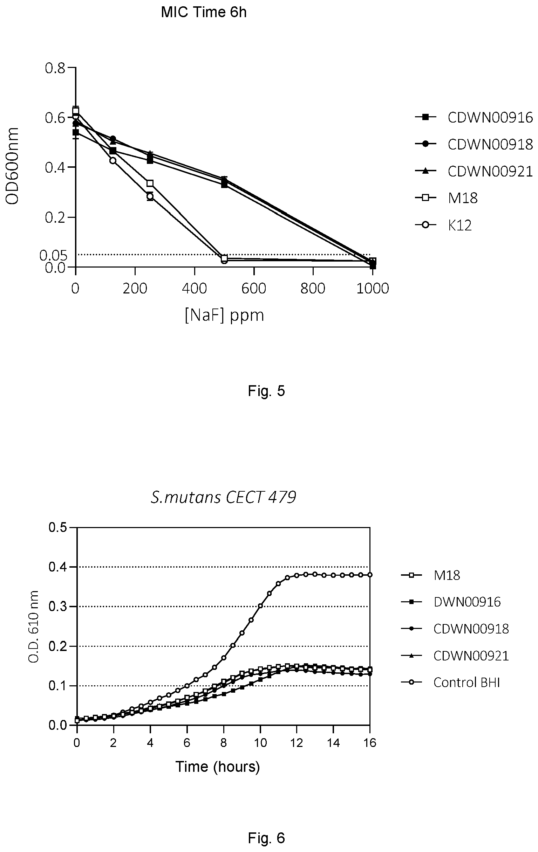

**Figure 5****:** Minimum inhibitory concentration (MIC) of the strains of the invention CDWN00916, CDWN00918, and CDWN00921 and the reference strains K12 and M18 against NaF-supplemented BHI medium at 37 °C after 6 hours of incubation. The data shown are the mean net O.D. values at 6 hours for each tested NaF concentration, as described in Figure 4.
**Figure 6****:** Inhibitory effect of the supernatants from the three *S. salivarius* strains of the invention on the growth of *Streptococcus mutans* CECT 479^{T}. Mean net OD values from two independent experiments are shown.

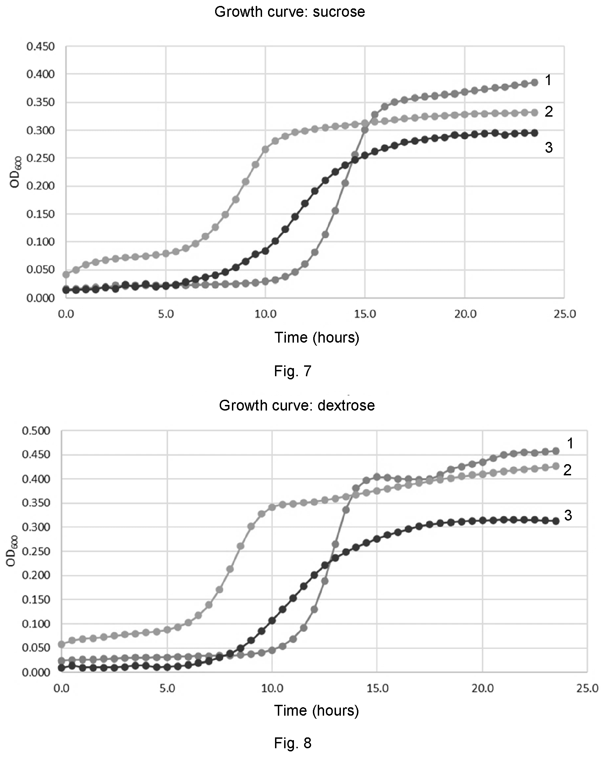

**Figure 7****:** Growth curve of the *S. salivarius* strains CDWN00916 (3), CDWN00918 (1), and CDWN00921 (2) in MRS medium supplemented with 0.2% sucrose (w/v), with cultures incubated statically at 37 °C for 48 hours. To quantify bacterial growth, OD₆₀₀ values were recorded.
**Figure 8****:** Growth curve of S. salivarius strains CDWN00916 (3), CDWN00918 (1), and CDWN00921 (2) in MRS medium supplemented with 0.2% dextrose (w/v) and incubated statically at 37 °C for 48 hours. To quantify bacterial growth, OD₆₀₀ values were recorded.

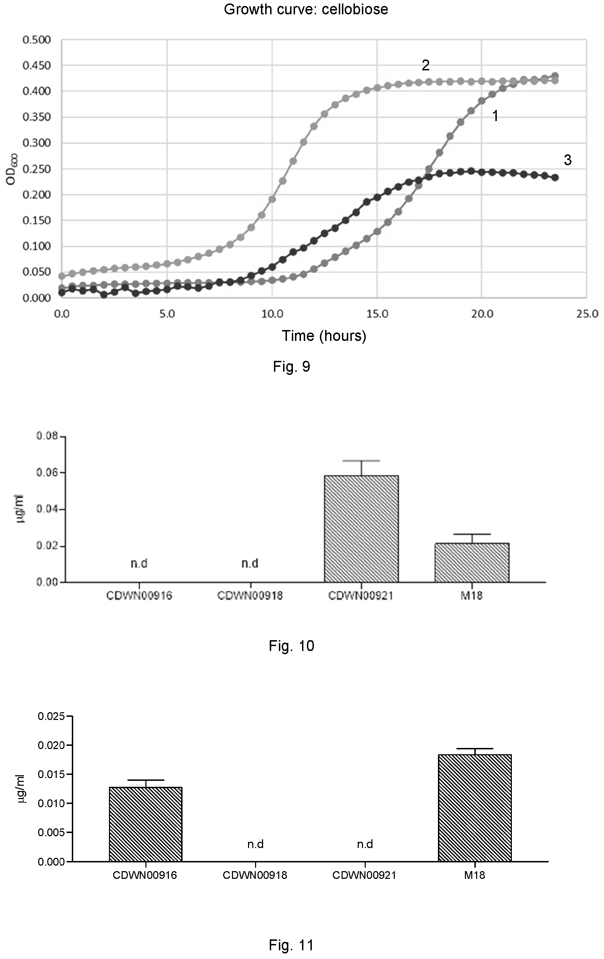

**Figure 9****:** Growth curves of *S. salivarius* strains CDWN00916 (3), CDWN00918 (1), and CDWN00921 (2) in MRS medium supplemented with 0.2% cellobiose (w/v), with cultures incubated statically at 37 °C for 48 hours. OD₆₀₀ values were recorded to quantify bacterial growth.
**Figure 10****:** Lysine concentration measured by UPLC in the supernatants of *S. salivarius* strains CDWN00916, CDWN00918, and CDWN00921 and the reference strain M18 after 6 hours of incubation in M9 minimal medium without amino acids. Mean values and standard deviation of biological duplicates are shown.
**Figure 11****:** Lysine concentration measured by UPLC in supernatants from *S. salivarius* strains CDWN00916, CDWN00918, and CDWN00921 and the reference strain M18 after 6 hours of incubation in modified M9 minimal medium. Mean values and standard deviations of biological duplicates are shown.

### Examples

### 1. Isolation and identification of the strains of the invention and evaluation of their ability to produce essential amino acids

Based on the study of microbial metabolism, the key genes and their metabolic functions involved in the biosynthetic pathways of several previously described essential amino acids have been identified. Table 1 provides a description of the key genes involved in the biosynthetic pathways of essential amino acids, as well as the function of the protein(s) they encode.

**Table 1. Key genes involved in the biosynthetic pathways of essential amino acids.**

| **Essential amino acid** | **Gen** | **Gen function** |
|---|---|---|
| Tryptophan | *trpA* | tryptophan synthase (α subunit) |
| Phenylalanine | *pheA* | chorismate mutase-prephenate dehydratase (CM-PDT) |
| Lysine | *lysA* | diaminopimelate decarboxylase |
| Histidine | *hisD* | histidinol dehydrogenase |
| Methionine | *metH*, *metE* | methionine synthase |

Taking into account the presence or absence of previously identified key genes in the biosynthetic pathways, the abundance of these pathways in oral bacteria was *in silico* quantified based on an analysis of their genomes (Table 2). According to the results obtained, the genus *Streptococcus* has the highest number of species associated with oral environments that possess essential amino acids biosynthetic pathways.

**Table 2: Abundance of key genes in the biosynthetic pathways of various essential amino acids in oral bacteria based on in silico analysis.**

| **Taxonomic group** | **Representative oral genres** | ***trpA*** | ***pheA*** | ***lysA*** | ***hisD*** | ***metH*** | ***metE*** |
|---|---|---|---|---|---|---|---|
| *Pseudomonadota* | *Neisseria* | ++ | +++ | ++ | ++ | ++ | ++ |
| | *Kingella* | + | + | ++ | -- | + | + |
| | *Haemophilus* | + | + | + | | + | + |
| *Campylobacterota* | *Campylobacter* | + | + | + | + | + | + |
| *Bacillota* | *Veillonella* | - | - | + | + | - | - |
| | *Streptococcus* | +++ | +++ | +++ | +++ | +++ | +++ |
| | *Granulicatella* | -- | -- | -- | -- | -- | -- |
| *Fusobacteriota* | *Fusobacterium* | - | - | - | - | + | - |
| | *Leptotrichia* | - | - | - | - | -- | - |
| *Bacteroidota* | *Prevotella* | + | + | + | + | + | - |
| | *Porphyromonas* | -- | -- | + | -- | -- | - |
| | *Capnocytophaga* | -- | + | + | -- | + | + |
| *Actinomycetota* | *Corynebacterium* | + | + | - | ++ | + | + |
| | *Actinomyces* | + | +++ | + | + | + | + |
| | *Rothia* | ++ | +++ | ++ | + | ++ | ++ |

Abundance of key genes involved in the synthesis of tryptophan, phenylalanine, lysine, histidine, and methionine in the most representative bacterial genera that make up the native oral microbiota. Frequencies are indicated as follows: (+) Present in non-oral species (or present in the mouth at a proportion <0.1%); (++) Present in oral species or in many species; (+++) Present in many oral species; (--) Absent/not detected in databases.

In this regard, we subsequently proceeded to perform a selective isolation of *Streptococcus* strains that are potential producers of EAs. and belong to species with isolates classified as GRAS. Briefly, 1-ml saliva samples were collected from volunteer subjects in sterile Eppendorf tubes. Sampling was performed first thing in the morning in healthy volunteers aged 20 to 50 who had neither eaten nor brushed their teeth since the previous night. After sample collection, serial dilutions were performed in PBS buffer, and the samples were inoculated onto Mitis Salivarius Agar (Sigma-Aldrich 01337-500G-F), a selective and differential medium for the isolation of *Streptococcus salivarius* and *S. mitis*. The Petri dishes were incubated at 37 °C under aerobic conditions for 72 hours. The grown colonies were purified using MRS culture medium (the composition of the MRS medium (Man, Rogosa, and Sharpe) was as follows: yeast extract (2.5%), sodium acetate (0.5%), MgSO₄·7H₂O (0.02%), MnSO₄·H₂O (0.005%) and 0.2% sugar.) and taxonomically identified through partial sequencing of the 16S ribosomal gene using the methodology described in Latorre-Pérez, A., Gimeno-Valero, H., Tanner, K., Pascual, J., Vilanova, C., & Porcar, M. (2021). A round trip to the desert: in situ nanopore sequencing informs targeted bioprospecting. Frontiers in Microbiology, 12, 768240. To determine the taxonomic affiliation of the isolates and their phylogenetically closest species, the online tool EzBioCloud (https://www.ezbiocloud.net/) was used. Among other microorganisms, strains CDWN00916, CDWN00918, and CDWN00921 were isolated and identified as members of the genus *Streptococcus* based on 16S rRNA gene similarity. Subsequently, the genomic sequences of the three strains were obtained following the methodology described in Vidal-Verdú, À., Molina-Menor, E., Pascual, J., Peretó, J., & Porcar, M. (2023). Gillisia lutea sp. nov., isolated from marine aluminum residues from the Mediterranean Sea. International Journal of Systematic and Evolutionary Microbiology, 73(8), 005957. Based on the genomic sequences, the taxonomic assignment of the three strains was analyzed in greater detail using the ANIb (Average Nucleotide Identity) genomic similarity index with the ANIb-based tool. The index was calculated using the JspeciesWS tool, and a phylogenomic tree was generated using the TYGS tool.

To assess the safety of the three strains, their profiles of virulence factors and resistance to clinically used antibiotics were analyzed *in silico*, following the recommendations of EFSA (2021) (European Food Safety Authority (EFSA). (2021). EFSA statement on the requirements for whole genome sequence analysis of microorganisms intentionally used in the food chain. EFSA Journal, 19(7), e06506.). The PathogenFinder and Abricate tools were used with the VFDB 2022 database to identify virulence factors, while the CARD tool was used with the "Perfect and Strict hits" criterion to detect antibiotic resistance. The results are shown in Tables 3, 4, and 5.

The *S. salivarius* strain identified as ATCC 7073 or NCTC 8618, among other designations, is a bacterial strain isolated from the blood of a patient with acute rheumatic fever; its use as an oral probiotic has been ruled out because it was isolated from a subject with a medical condition. Other names for this strain include the following: DSM 20560, ATCC 7073, NCTC 8618, JCM 5707, BCRC 17388, CCM 4046, CCUG 11878, CCUG 17825, CCUG 50207, CECT 805, CIP 102503, HAMBI 1716, LMG 11489, NCFB 1779, NCIMB 701779.

**Table 3. Species phylogenetically closest to the three strains included in the invention, based on 16S rRNA gene identity.**

| **Strain** | **Phylogenetically closest strain** | **Identity (%)** |
|---|---|---|
| CDWN00916 | *Streptococcus salivarius* subsp. *Salivarius* NCTC 8618(T) | 99.86 |
| CDWN00918 | *Streptococcus salivarius* subsp. *Salivarius* NCTC 8618(T) | 100 |
| CDWN00921 | *Streptococcus salivarius* subsp. *Salivarius* NCTC 8618(T) | 99.86 |

**Table 4. The phylogenetically closest species to the three strains included in the invention, based on the ANIb genomic index.**

| **Strain** | **Taxonomic classification** | **ANIb (%)** |
|---|---|---|
| CDWN00916 | *Streptococcus salivarius* subsp. *Salivarius* NCTC 8618(T) | 96.13 |
| CDWN00918 | *Streptococcus salivarius* subsp. *Salivarius* NCTC 8618(T) | 95.09 |
| CDWN00921 | *Streptococcus salivarius* subsp. *Salivarius* NCTC 8618(T) | 95.03 |

Index calculated with the JspeciesWS tool: Richter M, Rosselló-Móra R, Glöckner FO, and Peplies J (2015) JSpeciesWS: a web server for prokaryotic species circumscription based on pairwise genome comparison. Bioinformatics. 2015 Nov 16. pii: btv681 (https://jspecies.ribohost.com/jspeciesws/).

**Table 5. Genomic identity (%) among the three strains included in the invention, based on the ANIb genomic index (JspeciesWS).**

| | **CDWN00916** | **CDWN00918** | **CDWN00921** |
|---|---|---|---|
| **CDWN00916** | 100 | | |
| **CDWN00918** | 95.3 | 100 | |
| **CDWN00921** | 95.07 | 97.4 | 100 |

Based on the 16S rRNA gene analysis, the three strains covered by the invention-CDWN00916, CDWN00918, and CDWN00921-belong to the genus *Streptococcus* and are phylogenetically related to the species *Streptococcus salivarius* subsp. *salivarius*. The values obtained using the ANIb genomic index confirmed that the three strains belong to the species *Streptococcus salivarius* and are phylogenetically more closely related to the subspecies *Streptococcus salivarius* subsp. *salivarius* than to *Streptococcus salivarius* subsp. *thermophilus* (Tables 3, 4, and 5, and Figure 1). However, since the ANIb value is close to the threshold value, bioinformatic analysis of the genomes confirms that none of the three strains possesses genes encoding virulence factors or resistance to clinically used antibiotics. The absence of virulence factors and resistance genes in their genomes confirms that the three strains claimed in the invention are safe for use in humans.

Next, the amount of essential amino acids (EAs) produced and released into the medium by the three strains included in the invention was quantified. To do this, strains CDWN00916, CDWN00918, and CDWN00921 were cultured in minimal medium in the absence of amino acids, and the concentration of free amino acids in the supernatants was measured after 2 hours of incubation using nuclear magnetic resonance (NMR). It is important that the culture medium not contain amino acids, as this activates the biosynthetic pathways, which are strongly regulated by the presence of the metabolite.

Furthermore, the absence of amino acids allows for the quantification of those produced by the strain without contamination from the culture medium. The study included the state-of-the-art reference strain M18, which is marketed as an oral probiotic (Figure 2). The strains were grown in brain heart infusion (BHI) liquid medium in static culture at 37°C until reaching the stationary phase. The optical density (OD) of the culture was measured at an absorbance of 600 nm and adjusted to an OD value of 2 in 1 mL. The culture was washed by centrifugation at 4000 rpm for 5 minutes at room temperature, after which the supernatant was discarded and the pellet was resuspended in 1 mL of sterile 1x phosphate-saline buffer. The sample was centrifuged again as described above, and the pellet was resuspended in 1 mL of minimal medium (12.8 g/L Na₂HPO₄·7H₂O, 3 g/L KH₂PO₄, 0.5 g/L NaCl, 1 g/L NH₄Cl, 2 mM MgSO₄·7H₂O, 0.1 mM CaCl₂·2H₂O) supplemented with 1% (w/v) dextrose and 1% sucrose (w/v). The culture was then incubated statically at 37 °C for 2 hours in minimal medium supplemented with 1% (w/v) dextrose and 1% sucrose (w/v). After the incubation period, the culture was centrifuged at 13,000 rpm for 5 min at 4°C, and the supernatant was recovered and stored at -80°C until NMR analysis. The metabolites were analyzed using the ChenomX 8.6 software and TSPd4 in buffer as an internal standard (150 mM potassium phosphate, pH 7.4; 580.5 µM TSPd4; 10% D₂O; 0.05% sodium azide).

The use of NMR as an analytical technique eliminates the need for sample pretreatment; however, it is less sensitive than other analytical techniques and requires higher concentrations of the metabolite in the supernatant for its accurate identification and quantification. Therefore, the experimental conditions were optimized as described to achieve the most comprehensive characterization possible of the metabolite profile of commercial interest secreted by the selected isolates, and analysis was performed using complementary techniques such as liquid chromatography coupled with mass spectrometry (UPLC-QtoF-MS), using strain M18 as a reference and incubating *S. salivarius* cells in minimal culture medium for 6 hours (Figure 3). The samples were prepared as described in Figure 2. In summary, the cultures were grown to the stationary phase, and the OD was adjusted to a value of 2 in 1 mL. The culture was washed in phosphate-saline buffer, after which the pellet was resuspended in 1 mL of minimal medium supplemented with 1% (w/v) dextrose and 1% (w/v) sucrose and incubated statically at 37 °C for 6 hours. After the incubation period, the culture was centrifuged at 13,000 rpm for 5 min at 4°C, and the supernatant was collected and stored at -80°C. Prior to the analysis of free amino acids by UPLC, the proteins present in the sample were precipitated with ACN (2:1, v/v); the supernatant was then recovered and concentrated using a SpeedVac vacuum system. The samples were reconstituted in 0.3 mL of H₂O + 0.1% formic acid and analyzed on a UPLC-QtoF-MS platform.

Both NMR and UPLC analysis are routine procedures for an expert in the field, provided that the same experimental conditions described here are used-such as strain growth, the same type of equipment, standards, etc.

Based on the concentration of EAs produced by the strains, the number of colony-forming units (CFUs) of each of the three microorganisms that must be ingested daily to meet the EAs requirements of a 77-kg adult was calculated (Table 6). The method used to calculate the recommended daily intake (RDI) was described in *Protein and Amino Acid Requirements in Human Nutrition: Joint FAO/WHO/UNU Expert Consultation*, 2007 (https://apps.who.int/iris/handle/10665/43411), specifically based on the data in Table 23 of that report. The values are calculated based on the amino acid concentration detected in the supernatant and the count of viable cells at the end of the incubation period (6 h) for the same analyzed sample. The amount of amino acid produced per 1 CFU/1 h is calculated, and the number of CFUs required to meet the RDI is extrapolated. It should be noted that in the case of phenylalanine and tyrosine-as is also the case with methionine and cysteine-the DRI is grouped into a single value, since these amino acids are easily converted into one another.

**Table 6. CFUs of S. salivarius required to meet the daily essential amino acid needs of a 77-kg adult.**

| Final CFUs T=6h | **CFUs of *Streptococcus salivarius* to meet the RDI** | | | |
|---|---|---|---|---|
| Essential Amino acids (EAs) | CDWN00916 | CDWN00918 | CDWN00921 | M18 |
| Ile | 1,55x10⁹ | 1,70 x10⁹ | 5,31 x10⁷ | 1,57 x10¹⁰ |
| Leu | 2,06 x10⁹ | 2,22 x10⁹ | 7,10 x10⁷ | 2,10 x10¹⁰ |
| Lys | - | - | - | - |
| Phe + Tyr | 2,99 x10⁹ | 9,73 x10⁹ | 5,58 x10⁷ | 2,08 x10¹¹ |
| Thr | 5,63 x10⁹ | 3,12 x10¹⁰ | 4,83 x10⁸ | 1,28 x10¹¹ |
| Trp | 9,93 x10¹⁰ | - | - | 9,24 x10¹¹ |
| Val | 3,11 x10¹⁰ | 8,43 x10¹⁰ | 1,57 x10⁷ | 1,24 x10¹² |
| Met + Cys | 5,56 x10¹¹ | - | - | - |

As shown in Table 6, the *S. salivarius* strain CDWN00921 produces the highest amount of EAs; therefore, a lower dose of microorganisms is required to meet daily needs.

### 2. Fluoride tolerance assay: evaluation of the growth of Streptococcus salivarius strains CDWN00916, CDWN00918, CDWN00921, and reference strains M18 and K12 in the presence of increasing concentrations of sodium fluoride (NaF)

In this assay, the resistance of the bacterial strains to sodium fluoride (NaF)-a common ingredient in toothpastes used to prevent dental caries-was evaluated. The ability of these strains to resist NaF gives them a significant advantage by increasing their tolerance to oral hygiene procedures, which, in turn, allows them to colonize the oral cavity more effectively and for a longer duration. This benefit is reflected in the potential reduction in the number of probiotic strains required for administration and/or in the decreased frequency of administration needed.

### Growth curves and calculation of the minimum inhibitory concentration (MIC):

The strains were inoculated from glycerin stock into BHI (Brain Heart Infusion Broth) (Oxoid) and incubated aerobically at 37 °C for 3 days. Afterward, 10 ml of liquid BHI (Oxoid) pre-culture was inoculated with 1 CFU and incubated statically at 37 °C under aerobic conditions overnight. The following day, the OD₆₀₀ of the pre-culture was measured, and the inoculum was adjusted to an initial OD₆₀₀ of 0.1 in BHI. NaF (Sigma) solutions corresponding to 2000, 1000, 500, and 250 ppm in BHI medium were prepared. In a sterile 96-well flat-bottom plate, 100 µl of culture and 100 µl of the corresponding medium were dispensed in duplicate according to the experimental design, resulting in an initial OD600 of the culture of 0.05 and NaF concentrations of 1000, 500, 250, 125, and 0 ppm. The plate was incubated in a TECAN infinite^{®} M Plex microplate reader at 37 °C, and absorbance readings were taken at 600 nm every 30 minutes until the cultures generally reached the stationary phase at 6.5 hours (Fig. 4), following a standard protocol in the field. To calculate the minimum inhibitory concentration (MIC), the absorbance values at 6 h were plotted for each tested NaF concentration (Fig. 5).

These data confirm that the strains of the present invention (CDWN00916, CDWN00918, and CDWN00921) exhibit remarkable resistance to NaF, in contrast to conventional prior-art strains (M18 and K12). This translates into a substantially higher growth rate in the presence of fluoride. This increase in bacterial growth is evident even at normal fluoride exposure concentrations in the oral cavity, such as those found in toothpastes. In contrast, the prior-art strains grow more slowly or are even unable to grow under these conditions. The typical concentration in adult toothpastes is 1,500 ppm; saliva produces a dilution effect of approximately 1/3 to 1/4, resulting in a concentration of approximately 500 ppm to 375 ppm-a concentration at which the strains of the invention still grow, whereas this is the MIC value for the prior art strains M18 and K12. Fig. 5

Consequently, it can be concluded that the strains included in the patent-CDWN00916, CDWN00918, and CDWN00921-exhibit greater tolerance to NaF compared to the reference strains, facilitating their viability in the human oral cavity.

### 3. Evaluation of the antimicrobial activity of the Streptococcus salivarius strains CDWN00916, CDWN00918, and CDWN00921, and the reference strains M18 and K12

A beneficial characteristic of oral probiotic microorganisms is their ability to produce biomolecules with antimicrobial properties, capable of inhibiting the growth of pathogenic microorganisms, including those that play a crucial role in the formation of dental caries, such as *Streptococcus mutans*. The reference strain M18 is a probiotic that produces bacteriocins that act against the cariogenic bacterium *S. mutans*. In addition, it produces dextranase and urease enzymes, which help reduce dental plaque accumulation and acidification.

In this context, the ability of the strains of the present invention to exhibit an inhibitory capacity against oral pathogens equal to or greater than that observed in the prior-art reference strains was evaluated.

To conduct the study, a microplate growth inhibition assay was used, as described in López-López A, Camelo-Castillo A, Ferrer MD, Simon-Soro Á, Mira A. Health-Associated Niche Inhabitants as Oral Probiotics: The Case of Streptococcus dentisani. Front Microbiol. 2017 Mar 10;8:379. DOI: 10.3389/fmicb.2017.00379. PMID: 28344574; PMCID: PMC5344910., which allows for the evaluation of the ability of the supernatants from the strains to inhibit the pathogenic strain *S. mutans* CECT479 (Figure 6).

The strains of the invention and the *S. salivarius* M18 strain were grown to the stationary phase in BHI, and the cultures were centrifuged at 4000 rpm for 4 min at 4°C. The supernatant was passed through a 0.2 µm filter and then concentrated 10-fold using an IKA RV8 rotary evaporator. The concentrated supernatant was sterilized by 0.2 µm filtration. Next, *S. mutans* CECT 479 (type strain) was grown to the stationary phase in BHI, after which an initial OD₆₀₀ of 0.125 was adjusted in fresh BHI medium. In a 96-well microplate, 160 µl of the culture was dispensed per well, to which 40 µl of 10x concentrated supernatant or 10x concentrated BHI (as a control) was added, resulting in an initial OD₆₀₀ of 0.1 for the culture and a final concentration of 2x for the supernatants and BHI in the corresponding wells. The absorbance of the culture was then measured at 600 nm using a TECAN Infinite^{®} 200 PRO plate reader. Each condition was tested in triplicate, and measurements were taken every 30 minutes for 16 hours at 37°C.

The results showed that the inhibitory capacity of the three strains covered by this invention is similar to that of the prior-art reference strain.

### 4. Evaluation of the acidogenic activity of Streptococcus salivarius strains CDWN00916, CDWN00918, and CDWN00921, and reference strains M18 and K12

The objective of this experiment was to quantify the acidogenic activity of the *Streptococcus salivarius* strains of the invention using sugars typically consumed in the diet-specifically sucrose, dextrose, and cellobiose-in comparison with the reference strains *Streptococcus salivarius* M18 and K12. The acidogenic activity of these bacteria is undesirable, as it promotes the development of dental caries.

To evaluate acidogenic activity, the strains were grown in a culture medium optimal for the growth of *Streptococcus salivarius* (MRS medium) to which 0.2% (w/v) sugar was added as the sole source of carbon and energy. Three sugars typically consumed in the diet-sucrose, dextrose, and cellobiose-were tested. Consequently, a total of three medium variants were prepared, one containing each type of sugar tested.

Each of the *Streptococcus salivarius* strains of the invention was inoculated into each of the three MRS media at a final inoculum concentration equivalent to an OD₆₀₀ of 0.1. The growth kinetics of the strains with each of the different sugars were evaluated by measuring OD₆₀₀ at 30-minute intervals over 24 hours (Figures 7-9).

The acidogenic activity of the microorganisms was evaluated by measuring the pH of the culture media after the bacterial strains had grown at 37 °C under aerobic conditions in static culture for 48 hours. A PL-700PV pH meter was used to measure the pH. Each measurement was performed in duplicate. In addition, lactic acid production by each microorganism in the three culture media was quantified using the Y15 analyzer (Biosystems) with the Multical (Ref. 12818) and L-lactic Acid (Ref. 12802) kits from BioSystems. Lactic acid production in the culture media reflects the fermentative metabolism of the microorganisms. After culturing the microorganisms statically at 37 °C for 48 hours, they were centrifuged at 4700 rpm for 3 minutes in an Eppendorf Centrifuge 5910R, and the supernatant was filtered through nitrocellulose filters with a pore size of 0.2 µm prior to measurement with the Y15 analyzer. As a negative control, the culture medium supplemented with the corresponding sugar and without any inoculated microorganisms was tested.

Among the three *Streptococcus salivarius* strains under the scope of the invention, strain CDWN00921 has the shortest generation time, regardless of the sugar used, reaching the stationary phase faster than the others. In contrast, strain CDWN00918 has the longest generation time; however, it reaches a cell density comparable to that of strain CDWN00921 in the stationary phase. Strain CDWN00916 achieves the lowest cell density after growth (Figures 7-9).

Strain CDWN00918 produces the lowest level of medium acidification and the lowest amount of lactic acid compared to the other *Streptococcus salivarius* strains tested, including the prior art strains M18 and K12 (see Table 4). Strains CDWN00916 and CDWN00921 exhibit acidification and lactic acid production values similar to those of the prior-art strains when using sucrose and dextrose. However, with cellobiose, strain CDWN00916 exhibits values comparable to those of strain CDWN00918 and, therefore, higher than those of the prior art strains. As expected, a positive correlation is observed between the pH of the medium after growth and the concentration of lactic acid produced. These results indicate that strain CDWN00918 is less acidogenic than the prior art strains and, consequently, is the least likely to promote the growth of pathogenic bacteria involved in cariogenic processes, regardless of the sugar tested. In the case of cellobiose, CDWN00916 also exhibits lower acidogenic activity than the prior art strains (see Tables 4 and 5).

**Table 4. Acidification values (pH) of the media after 48 hours of incubation in culture media containing each of the sugars**

| **Strain** | **MRS + 0,2% sucrose** | **MRS + 0,2% dextrose** | **MRS + 0,2% cellobiose** |
|---|---|---|---|
| **CDWN00918** | 5,19 ± 0.03 | 5,30 ± 0.01 | 5,14 ± 0.03 |
| **CDWN00916** | 4,84 ± 0.08 | 4,92 ± 0.03 | 5,21 ± 0.01 |
| **CDWN00921** | 4,92 ± 0.01 | 4,95 ± 0.00 | 4,83 ± 0.03 |
| **M18** | 4,87 ± 0.03 | 4,93 ± 0.03 | 4,99 ± 0.03 |
| **K12** | 4,88 ± 0.06 | 4,94 ± 0.01 | 4,98 ± 0.01 |
| **Negative Control** | 5,39 ± 0.01 | 6,64 ± 0.04 | 6,64 ± 0.06 |

| | | | |
|---|---|---|---|
| Mean values and their corresponding standard deviation (n =3) . | | | |

**Table 5. Lactic acid concentration values (g/L) produced by the microorganisms using each of the tested sugars after 48 hours of incubation.**

| **Strain** | **MRS + 0,2% sucrose** | **MRS + 0,2% dextrose** | **MRS + 0,2% cellobiose** |
|---|---|---|---|
| **CDWN00918** | 2,00 ± 0,03 | 1,73 ± 0,06 | 0,31 ± 0,01 |
| **CDWN00916** | 3,62 ± 0,09 | 3,13 ± 0,02 | 0,70 ± 0,13 |
| **CDWN00921** | 2,08 ± 0,04 | 1,65 ± 0,09 | 1,54 ± 0,08 |
| **M18** | 3,35 ± 0,06 | 2,98 ± 0,07 | 1,93 ± 0,07 |
| **K12** | 3,33 ± 0,01 | 2,84 ± 0,05 | 1,86 ± 0,13 |
| **Negative Control** | 0,11 ± 0,01 | 0,11 ± 0,01 | 0,12 ± 0,01 |

| | | | |
|---|---|---|---|
| Mean values and their corresponding standard deviation (n =3) . | | | |

### 5. Comparison of the genomes of the strains of the invention CDWN00916, CDWN00918, and CDWN00931 with strain NCTC 8618

The genomes of strains CDWN00916 (GenBank ID JAXKQD000000000), CDWN00918 (GenBank ID JAXKQE000000000), CDWN00921 (GenBank ID JAXKLG000000000), and NCTC 8618 (ENA ID LR134274.1) were annotated using the BV-BRC Genome Annotation Service (Olson, R. D., Assaf, R., Brettin, T., Conrad, N., Cucinell, C., Davis, J. J., ... & Stevens, R. L. (2023). Introducing the bacterial and viral bioinformatics resource center (BV-BRC): a resource combining PATRIC, IRD, and ViPR. Nucleic Acids Research, 51(D1), D678-D689), using the RAST (RASTtk) tool [Aziz, R. K. et al. The RAST Server: rapid annotations using subsystems technology. BMC Genomics 9, 75 (2008)]. Subsequently, the pangenome of the strains was analyzed using the tool available on BV-BRC, The Protein Family Sorter (PFS), by selecting the PATRIC genus-specific families (PLfams) option.

It was observed that the beta-galactosidase (EC 3.2.1.23; PLF_1301_00011912) and lactose/galactose permease (PLF_1301_00015661) genes are present in the three strains of the invention but absent in the reference strain. Surprisingly, these results suggest a possible additional advantage of the strains of the invention, namely improved digestion of milk and dairy products, which would help people who are lactose intolerant.

### 6. Quantification of Lysine in Supernatants in Minimal Medium Without Amino Acids

This protocol is identical to that used in Example 1 and Fig. 3. The amount of lysine (lys) produced and released into the medium by the three strains included in the invention-CDWN00916, CDWN00918, and CDWN00921-was quantified. The study included the prior-art reference strain M18, which is marketed as an oral probiotic. The strains were grown in brain-heart infusion (BHI) liquid medium in static culture at 37°C until reaching the stationary phase. The optical density (OD) of the culture was measured at an absorbance of 600 nm and adjusted to an OD value of 2 in 1 mL. The culture was washed by centrifugation at 4000 rpm for 5 minutes at room temperature, after which the supernatant was discarded and the pellet was resuspended in 1 mL of sterile 1x phosphate-saline buffer. The sample was centrifuged again as described above, and the pellet was resuspended in 1 mL of M9 minimal medium (12.8 g/L Na₂HPO₄·7H₂O, 3 g/L KH₂PO₄, 0.5 g/L NaCl, 1 g/L NH₄Cl, 2 mM MgSO₄·7H₂O, 0.1 mM CaCl₂·2H₂O) supplemented with 1% (w/v) dextrose and 1% (w/v) sucrose, after which it was incubated statically for 6 hours. After the incubation period, the culture was centrifuged at 13,000 rpm for 5 min at 4°C, and the supernatant was collected and stored at -20°C. Prior to the analysis of free amino acids by UPLC, the proteins present in the sample were precipitated with ACN (2:1, v/v); the supernatant was then recovered and concentrated using a SpeedVac vacuum system. The samples were reconstituted in 0.3 mL of H₂O + 0.1% formic acid and analyzed on a UPLC-QtoF-MS platform. The results are shown in Fig. 10.

### 7. Quantification of Lysine in Supernatants in Lysine-Free Supplemented Medium

The amount of lysine (lys) produced and released into the medium by the three strains included in the invention-CDWN00916, CDWN00918, and CDWN00921-was quantified. The prior art reference strain M18 was included in the study. The strains were grown in brain-heart infusion (BHI) liquid medium in static culture at 37°C until reaching the stationary phase. The optical density (OD) of the culture was measured at an absorbance of 600 nm and adjusted to an OD value of 2 in 1 mL. The culture was washed by centrifugation at 4000 rpm for 5 minutes at room temperature, after which the supernatant was discarded and the pellet was resuspended in 1 mL of sterile 1x phosphate-saline buffer. The sample was centrifuged again as described above, and the pellet was resuspended in 1 mL of modified M9 minimal medium (12.8 g/L Na₂HPO₄·7H₂O, 3 g/L KH₂PO₄, 0.5 g/L NaCl, 1 g/L NH₄Cl, 2 mM MgSO₄·7H₂O, 0.1 mM CaCl₂·2H₂O) supplemented with 1% (w/v) dextrose, amino acids (except lysine; Table 1), and 2X MEM vitamins (Gibco^{™}), after which it was incubated statically for 6 hours. After the incubation period, the culture was centrifuged at 13,000 rpm for 5 min at 4°C, and the supernatant was collected and stored at -20°C. Prior to the analysis of free amino acids by UPLC, the proteins present in the sample were precipitated with ACN (2:1, v/v); the supernatant was then recovered and concentrated using a SpeedVac vacuum system. The samples were reconstituted in 0.3 mL of H₂O + 0.1% formic acid and analyzed on a UPLC-QtoF-MS platform. The results are shown in Fig. 11

**Table 6: List of amino acids and final concentrations (mg/L) in the modified M9 medium.**

| Amino acid | | Concentration in the medium (mg/L) |
|---|---|---|
| Tryptophan | L-trp | 100 |
| Histidine | L-his | 100 |
| Valine | L-val | 150 |
| Methionine | L-met | 100 |
| Isoleucine | L-ile | 150 |
| Leucine | L-leu | 150 |
| Threonine | L-thr | 150 |
| Phenylalanine | L-phe | 100 |
| Arginine | L-arg | 100 |
| Glycine | L-gli | 100 |
| Cysteine | L-cys | 50 |
| Proline | L-pro | 150 |
| Serine | L-ser | 100 |
| Alanine | L-ala | 100 |
| Tyrosine | L-tyr | 100 |
| Aspartic acid | L-asp | 150 |
| Glutamic acid | L-glu | 150 |

### List of sequences

**SEQ ID NO: 1** Sequence of the 16S rRNA ribosomal gene from strain CDWN00916
**SEQ ID NO: 2** Sequence of the 16S rRNA ribosomal gene from strain CDWN00918
**SEQ ID NO: 3** Sequence of the 16S rRNA ribosomal gene from strain CDWN00921

## Claims

1. An oral probiotic strain of *Streptococcus salivarius* that:
(a) is *Streptococcus salivarius* CDWN00916, deposited in the Spanish Type Culture Collection (CECT) under accession number CECT 30898,
(b) is *Streptococcus salivarius* CDWN00918, deposited in the Spanish Type Culture Collection (CECT) under accession number CECT 30899, or
(c) is *Streptococcus salivarius* CDWN00921 deposited in the Spanish Type Culture Collection (CECT) under accession number CECT 30900,
that has a minimum inhibitory concentration (MIC) to fluoride ions of at least 500 ppm

2. The strain according to the preceding claim, that has the properties of an oral probiotic: production of essential amino acids and antimicrobial activity.

3. The strain according to the preceding claim, wherein the essential amino acids are selected from one or more of the following: isoleucine, leucine, lysine, methionine, phenylalanine, threonine, tryptophan, and valine.

4. The strain according to any of the preceding claims, wherein resistance to fluoride comprises an MIC of at least 550 ppm of fluoride ion.

5. The strain according to any of the preceding claims, wherein it is in the form of a postbiotic.

6. An active extract obtained from one or more of the strains defined in any of claims 1 to 5.

7. A probiotic formulation for oral administration comprising one or more of the strains defined in any of claims 1 to 5 and/or the active extract of the preceding claim.

8. The formulation according to the preceding claim, comprising:
at least one pharmaceutically acceptable carrier, diluent, and/or excipient and/or
at least one active agent.

9. The formulation according to any of the preceding claims 7 or 8, wherein the active agent is at least one other different strain defined according to any one of claims 1 to 5 and/or another strain of *S. salivarius*.

10. The formulation according to any of the preceding claims 7 to 9, comprising the *S. salivarius* strain CECT 30898 and CECT 30900.

11. The formulation according to any of the preceding claims 7 to 10, which is included in a food, beverage, and/or nutraceutical.

12. The formulation according to any of the preceding claims 7 to 11, comprising approximately 10⁵ to 10¹² CFU, preferably 10⁸ to 10¹⁰ CFU, of one or more of the strains defined in any one of claims 1 to 5.

13. Use of one or more of the strains as defined in any of the preceding claims 1 to 5 and/or the formulation defined in any of claims 7 to 12 for the production of essential amino acids.

14. A strain as defined in any of the preceding claims 1 to 5, or an active extract according to claim 6, or a formulation according to any of the preceding claims 7 to 12, for use as a medicament.

15. A strain as defined in any of the preceding claims 1 to 5, or an active extract according to claim 6, a formulation according to any of the preceding claims 7 to 12, or a strain for use as a medicament according to claim 14, for use in the prevention or treatment of an oral disease.

16. The strain for use according to the preceding claim, wherein the oral disease is selected from dental caries and periodontal disease.

17. The strain for use according to the preceding claim, wherein the dental caries is caused at least in part by *S. mutans*.

18. A strain as defined in any of the preceding claims 1 to 5, or an active extract according to claim 6, or a formulation according to any of the preceding claims 7 to 12, for use in the prevention or treatment of dysbiosis or an imbalance of the oral microbiota, including dental caries, periodontitis, and halitosis.
